# EUROPEAN PATENT SPECIFICATION

(11) **EP 0 357 760 B1**
(45) Date of publication and mention of the grant of the patent: **26.05.1993**
(21) Application number: 89903607.3
(22) Date of filing: 10.03.1989
(51) Int. Cl.: A61C 5/00

(54) **DENTAL LASER**
DENTALER LASER
LASER DENTAIRE

(30) Priority: 14.03.1988 US 167739
(43) Date of publication of application: 14.03.1990
(73) Proprietor: AMERICAN DENTAL TECHNOLOGIES, INC., Michigan 48084 (US)
(72) Inventor: Vassiliadis, Arthur, Sunnyvale, CA 94089 (US); Myers, William, D., Birmingham, MI 48010 (US); Myers, Terry D., Farmington Hills, MI 48018 (US)
(74) Representative: Bryer, Kenneth Robert
(86) International application number: US8900984
(87) International publication number: WO8908432

(56) References cited:
- EP-A- 0 073 617
- US-A- 3 978 427
- US-A- 4 141 362
- US-A- 4 273 535
- US-A- 4 503 853
- US-A- 4 521 194
- LASER APPLICATIONS IN MEDICINE AND RIOLOGY, Vol. 2 issued 1974 (Durham, North Carolina) Ralph H. Stern, "Denistry And The Laser," see pages 361-388.

## Description

### Background of the Invention

### I. Field of the Invention

The present invention relates generally to lasers and, more particularly, to a dental laser which is particularly suited for removing dental enamel and dentin, and for cutting soft tissues.

### II. Description of the Prior Art

Diseased dental enamel and diseased dentin have traditionally been removed by the dentist by a mechanical drill. On the other hand, diseased soft tissue in the mouth is traditionally removed by cutting with a scalpel, scissors and the like. Both of these methods are uncomfortably painful to the patient, and the results produced have certain drawbacks.

There have, however, been a number of previously known experiments in which teeth have been subjected to laser radiation to determine the alteration, if any, of the physical or chemical properties of the dental enamel. These tests have shown that the hydroxyapatite crystals, that form the enamel, fuse somewhat at the surface when lased and render the enamel more impervious to acids of a type which cause tooth decay.

These previous studies have, however, also concluded that the laser cannot be used to remove tooth decay since the power level necessary for the laser to form an opening in the enamel also significantly heats up the tooth and can damage the dental pulp and kill the tooth nerve. For this reason, dental lasers have not been previously used to remove tooth decay or dentin. The lasers that were used previously were operated in different modalities and used different laser materials and were of different wavelengths than those proposed herewith.

In our prior U.S. Patent No. 4,521,194, we disclose a method for removing incipient carious lesions from the teeth. Such lesions are essentially a surface defect formed on the tooth so that their removal does not require forming an opening in the dental enamel. Furthermore, in that application it was believed that a laser should not be used to form an opening in the dental enamel and dentin for the above-discussed reasons.

### Summary of the Present Invention

The present invention provides an apparatus for removing decay, dental enamel and dentin. The same laser apparatus can also be used to cut soft tissues by laser.

In brief, the present invention comprises a laser and means for activating the laser so that the laser produces a pulsed output having a beam diameter in the range of 50-2,000 microns, a pulse duration in the range of several picoseconds to several milliseconds, an energy level of between .1-100 millijoules per pulse, and pulse repetition rate of from 1 to 200 pulses per second.

In another configuration, a small tip is used to concentrate the radiation so that effective soft tissue cutting can be made. The laser also produces an output pulse having a wavelength in the range of 2.0 - 3.0 microns. These wavelengths have been shown to be particularly effective for removing dental enamel and dentin. It has also been shown that these wavelengths are highly absorbed by soft tissue in the mouth. The exposures to enamel can he made without significantly heating the tooth or damaging the pulp and the tooth nerve. Furthermore, it has been found that a laser utilizing this wavelength together with the above-mentioned characteristics or laser parameters is capable of effectively and efficiently forming an opening in dental enamel rather than fusing the enamel as taught by the previous studies.

In one form of the invention, the laser is preferably an Erbium doped ytterium-aluminum-garnet (YAG) laser having a wavelength of substantially 2.94 microns. In the second embodiment of the invention, the laser is preferably a Holmium doped crystal laser, having a wavelength of substantially 2.06 microns. Other types of pulsed lasers can, however, alternatively be used.

In use, the laser is repeatedly pulsed until the decay, enamel, dentin or tissue is eradicated. Furthermore, in practice, it has been found that the apparatus of the present invention removes decay, enamel and dentin painlessly.

### Brief Description of the Drawing

A better understanding of the present invention will be had upon reference to the following detailed description when read in conjunction with the accompanying drawing, wherein like reference characters refer to like parts throughout the several views, and in which:
FIG. 1 is a perspective view of a preferred embodiment of the present invention;
FIGS. 2 and 3 are diagrammatic views illustrating the operation of the preferred embodiment of the present invention;
FIG. 4 is a cross-sectional view taken substantially along 4-4 in FIG. 3;
FIG. 5 is a view similar to FIG. 4 but showing a different type of decay; and
FIG. 6 is a partial side view showing a modification of the present invention.

### Detailed Description of a Preferred Embodiment of the Invention

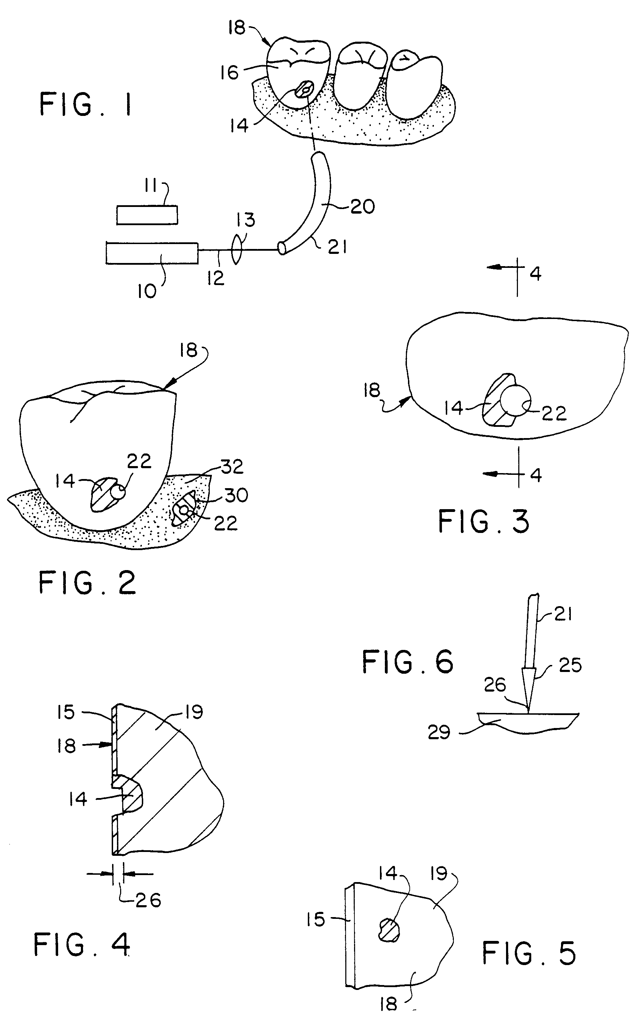

With reference first to FIG. 1, a preferred embodiment of the apparatus of the present invention is thereshown and comprises a laser 10 which, upon activation, generates a laser beam 12. A conventional means 11 is employed to energize or activate the laser 10. The laser beam 12 is focused into a fiber by a lens 13.

The laser 10 produces a pulse output having a beam with a pulse duration in the range of several picoseconds to several milliseconds and an energy of .1-100 millijoules per pulse, and pulse repetition rates of from 1 to 200 pulses per second. In addition, the laser 10 has a wavelength of between 2.0 and 3.0 microns which has been shown to be particularly effective in eradicating dental enamel and dentin. The laser beam diameter at the target is between 50-2,000 microns. It has also been shown that these wavelengths are very effective in cutting soft tissues.

Although any type of laser can be employed, in one form of the invention, the laser is an Erbium doped ytterium-aluminum-garnet (YAG) laser having a wavelength of substantially 2.94 microns. Alternatively, the laser 10 is a Holmium crystal laser having a wavelength of 2.06 microns. In both cases, these wavelengths have proven to be particularly effective for enamel interaction and thus for the eradication of enamel. Enamel 15, of course, is much harder than dentin so that the lasers which eradicate the enamel also are effective in eradicating dentin and decay.

With reference now to FIG. 1, 2 and 4, the laser 10 is employed to remove tooth decay 14 in the tooth 18, which, as shown in FIG. 4, has invaded the tooth dentin 19. A laser output beam 12 is aimed at decay 14 through any conventional delivery system 20, such as an optical fiber 21. A lens 1.3 is used to focus the output from the laser 10 into one end of the fiber 21 while the other end of the fiber 21 is focussed on the target, i.e. enamel 15, dentin 19, decay and/or soft tissue. A single optical fiber, furthermore, has proven particularly effective for use in dental applications since it may be bent easily to direct or deliver the laser beam to the desired location within the small area of the human mouth.

Other aiming systems can, of course, alternatively be used. For example, as best shown in FIG. 6, a contacting tip 25 may be employed with the optical fiber 21. The tip 25 is typically conical or frustoconical in shape and concentrates the laser energy at its apex 27. The apex 27 then contacts the target 29 (enamel, dentin, decay or soft tissue) in operation. Furthermore, the tip 25 has proven particularly effective for removing soft tissue.

With reference now to FIGS. 2-4, upon activation of the laser 10 by the means 11, the laser eradicates by vaporization the tooth decay 14 and/or enamel and/or dentin 19 in the area 22 of the laser beam impingement into a depth 26 (FIG. 4) into the decay 14 in the dentin 19. Thereafter, the laser is reaimed through the delivery system 20 to the remaining portions of the tooth decay 14 and reactivated until the entire decay 14 is eradicated or obliterated from the tooth.

The energy level of the laser is preferably adjustable to produce energy power levels of between .1 and 100 millijoules which correspondingly varies the depth 26 of the decay 14 obliterated by the laser 10, or the amount of enamel or dentin that is removed. For interacting with tooth decay, which has invaded a tooth to only a relatively shallow depth, relatively low laser energy is used. On the other hand, to remove enamel and dentin, then high energy levels need to be used. It is for this reason that a wide range of energy levels need to be available.

The precise phenomenon which occurs when the tooth is lased and the decay obliterated is not precisely understood due primarily to the extremely short time period involved during the lasing operation. It has been found, however, that both the dental enamel and the dentin is obliterated without signficantly heating the tooth and thus without damaging the nerve.

With reference now to FIG. 5, in some situations the decay 14 is inside the tooth 18 and is surrounded by healthy dentin 19 and healthy enamel 15. In this case, it is necessary to remove both the healthy enamel 15 and healthy dentin 19 before the decay can be removed by the laser. This has previously been done by drilling.

As best shown in FIG. 2, the laser of the present invention also effectively removes disease 30 from soft tissue 32, such as gum tissue. In order to remove soft tissue, a special tip may he used. The laser uses low energy levels, but high repetition rates of the same wavelength as previously described, and the tip is placed at the diseased portion of the soft tissue and a cutting action is initiated by the means 11. Once activated, the disease portion 30 of the soft tissue 32 is cut away, thus removing the disease and, simultaneously, sterilizing the soft tissue. Repeated activations of the laser may be required to completely eradicate the diseased portion 30 of the soft tissue and the removal of the diseased portion 30 is painlessly accomplished.

Having described my invention, many modifications thereto will become apparent to those skilled in the art to which it pertains without deviation from the spirit of the invention as defined by the scope of the appended claims.

## Claims

1. Apparatus for removing dental enamel, dentin and/or soft tissue comprising:
a laser,
means for activating said laser so that said laser produces a pulsed output having a wavelength of between 2.0 and 3.0 microns, a beam diameter in the range of 50-2000 microns, a pulse duration in the range of several picoseconds to several milliseconds and an energy of .1-100 millijoules,
means for delivering said pulse to dental enamel, dentin and/or soft tissue to thereby eradicate the dental enamel, dentin and/or soft tissue.

2. The invention as defined in claim 1 wherein the laser has a pulse repetition rate of 1 to 200 pulses per second.

3. The invention as defined in claim 1 wherein said laser is a yttrium-aluminum-garnet (YAG) laser.

4. The invention as defined in claim 3 wherein said laser is a Holmium doped crystal laser.

5. The invention as defined in claim 3 wherein said laser is an Erbium doped laser.

6. The invention as defined in claim 5 wherein said laser has a wavelength of substantially 2.94 microns.

7. The invention as defined in claim 4 wherein said laser has a wavelength of substantially 2.06 microns.

8. The invention as defined in claim 1 wherein said delivering means comprises means for focussing said pulse on one end of an optical fiber strand.

9. The invention as defined in claim 8 and comprising a contact tip adjacent the other end of said strand.

10. The invention as defined in claim 9 wherein said tip is conical in shape.

## Patentansprüche

1. Vorrichtung zum Entfernen von Zahnschmelz, Dentin und/oder weichem Gewebe, mit:
einem Laser,
einer Einrichtung zum derartigen Betreiben des Lasers, daß dieser einen gepulsten Ausgangsstrahl mit einer Wellenlänge zwischen 2,0 und 3,0 Mikrometer, einem Strahldurchmesser in einem Bereich von 50-2000 Mikrometer, einer Impulsdauer im Bereich von mehreren Picosekunden bis zu mehreren Millisekunden und eine Energie von .1-100 Millijoule aufweist,
einer Zuführungseinrichtung zum Zuführen des Impulses zu dem Zahnschmelz, Dentin und/oder weichen Gewebe, um so den Zahnschmelz, das Dentin und/oder das weiche Gewebe zu eradizieren.

2. Die in Anspruch 1 definierte Erfindung, wobei der Laser eine Impulsfrequenz von 1 bis 200 Impulsen pro Sekunde aufweist.

3. Die in Anspruch 1 definierte Erfindung, wobei der Laser ein Yttrium-Aluminium-Granat-Laser (YAG) ist.

4. Die in Anspruch 3 definierte Erfindung, wobei der Laser ein mit Holmium dotierter Kristallaser ist.

5. Die in Anspruch 3 definierte Erfindung, wobei der Laser ein mit Erbium dotierter Laser ist.

6. Die in Anspruch 5 definierte Erfindung, wobei der Laser ein Wellenlänge von im wesentlichen 2,94 Mikrometer ausstrahlt.

7. Die in Anspruch 4 definierte Erfindung, wobei der Laser eine Wellenlänge von im wesentlichen 2.06 Mikrometer ausstrahlt.

8. Die in Anspruch 1 definierte Erfindung, wobei die Zuführungseinrichtung eine Einrichtung aufweist, um den Impuls an einem Ende eines optischen Faserstrangs zu fokussieren.

9. Die in Anspruch 8 definierte Erfindung, wobei eine Kontaktspitze vorgesehen ist, welche an dem anderen Ende des Strangs angeordnet ist.

10. Die in Anspruch 9 definierte Erfindung, wobei die Spitze eine konische Form aufweist.

## Revendications

1. Appareil d'élimination d'émail dentaire, de dentine et/ou de tissus tendres comprenant :
un laser
des moyens pour activer le laser précité de telle sorte que celui-ci produise une impulsion de sortie ayant une longueur d'onde comprise entre 2,0 et 3,0 micromètres, un diamètre de faisceau de 50 à 2.000 micromètres, une durée d'impulsion de plusieurs picosecondes à plusieurs millisecondes et une énergie de 0,1 à 100 millijoules,
des moyens pour délivrer l'impulsion précitée à de l'émail dentaire, de la dentine et/ou à des tissus tendres pour éradiquer de la sorte l'émail dentaire, la dentine et/ou les tissus tendres.

2. Invention selon la revendication 1, dans laquelle le laser précité a une fréquence de répétition d'impulsions de 1 à 200 impulsions à la seconde.

3. Invention selon la revendication 1, dans laquelle le laser précité est un laser à yttrium-aluminium-grenat (YAG).

4. Invention selon la revendication 3, dans laquelle le laser précité est un laser de crystal dopé à l'holmium.

5. Invention selon la revendication 3, dans laquelle le laser précité est un laser dopé a l'erbium.

6. Invention selon la revendication 5, dans laquelle le laser précité a une longueur d'onde qui est sensiblement de 2,94 micromètres.

7. Invention selon la revendication 4, dans laquelle le laser précité a une longueur d'onde qui est sensiblement de 2,06 micromètres.

8. Invention selon la revendication 1, dans laquelle les moyens de délivrance précités comprennent des moyens pour focaliser l'impulsion précitée sur une extrémité d'un brin de fibres optiques.

9. Invention selon la revendication 8 comprenant par ailleurs une pointe de contact à proximité de l'autre extrémité du brin précité.

10. Invention selon la revendication 9, dans laquelle la pointe précitée a une forme conique.
